# EUROPEAN PATENT APPLICATION

(11) **EP 2 520 644 A1**
(43) Date of publication of application: **07.11.2012**
(21) Application number: 11382132.6
(22) Date of filing: 06.05.2011
(51) Int. Cl.: C12N 1/20, A23L 1/03, A61K 35/74, C12R 1/225

(54) **Lactobacillus fermentum CECT 7472 strain with probiotic properties**

(71) Applicant: Biotmicrogen, S.L., 18100 Armilla (ES); Cajamar Caja Rural, Sociedad Cooperativa De Crédito, 04006 Almería (ES); Sociedad Cooperativa Andaluza la Pastora de Taberno, 04692 Taberno (ES); Sociedad Cooperativa Andaluza Los Filabres, 04271 Sorbas (ES)
(72) Inventor: Lasserrot Cuadrado, Agustín, 18199 Cajar (ES); Ruiz Løpez, Maria Dolores, 18014 Granada (ES); Ruiz Bravo Løpez, Alfonso, 18150 Gojar (ES)
(74) Representative: ZBM Patents

(57) **Abstract**

The invention relates to a Lactobacillus fermentum strain with probiotic properties and enhanced activity against enteropathogens. The strain is Lactobacillus fermentum CECT 7472, which may be used as an ingredient in edible products, while maintaining its beneficial gastrointestinal health properties.

## Description

The present invention relates to the fields microbiology and food technology and particularly, to a novel probiotic strain of Lactobacillus fermentum for use in the benefit of health.

### BACKGROUND ART

Listeria monocytogenes is one of the causative agents of potentially fatal foodborne infections. The clinical symptoms go from febrile gastroenteritis to severe invasive forms including meningitis, encephalitis, abortions, septisemia, and perinatal infections. This bacterium is a Gram-positive facultative pathogen, which evolved multiple strategies to face the innate defence mechanisms of the host. L. monocytogenes may directly contaminate milk as a consequence of listerial mastitis and is a bacterium that can be found in cheese, yogurt and other foods, since it can resist the ripening process of most of these milk derivatives.

Due to the great mortality percentages (meningitis, 70 %; septicemia, 50 %; and perinatal infections, 80 %), there is a growing interest in finding good therapeutic strategies against listeriosis (infection caused by L. monocytogenes).

Nowadays, listeriosis is quickly treated with parenteral penicillin or ampicillin, and for penicillin-allergenic patients, trimethoprim-sulfamethoxazole is used. All these therapeutical approaches are based on pharmaceutical compositions, which apart from the side-effects associated to the same, tend not to be beneficial for the patient.

On the other hand, Yersinia enterocolitica, which is a Gram-negative cocobacillus-shaped bacterium, causes severe diarrhoea in humans, along with Peyer's patch necrosis, chronic lymphadenopathy, and hepatic or splenic abcesses. Additional symptoms may include entero-colitis, fever, mesenteric adenitis, erythema nodosum and acute terminal ileitis, which may be confused with appendicitis or Crohn's disease. Because Yersinia is a siderophilic (iron-loving) bacteria, people with hereditary hemochromatosis (a disease resulting in high body iron levels) are more susceptible to infection with Yersinia (and other siderophilic bacteria). In fact, the most common contaminant of stored blood is Y. enterocolitica.

The treatment of Y. enterocolitica infections often requires aggressive antibiotic therapy, typically involving ciprofloxacin, chloramphenicol, ampicillin, and polymyxin.

Lactobacillus fermentum is a Gram positive lactic acid bacteria (LAB), commonly found in many fermented food products, especially dairy products. L. fermentum strains are especially suitable for the industrial preparation of fermented food products thanks to their good survival rate through the industrial process and conservation period, as well as their high acidification profile and good organoleptic properties. Some strains of L. fermentum are also considered as probiotics. Probiotics are live microorganisms which, when administered in adequate amounts, provide health benefit to the host. To be termed as probiotic, the bacteria must fulfil several requirements related to their lack of toxicity, their resistance to acid environments and bile salts, their viability in reaching the lower gastrointestinal tract live (GIT), and their adhesion to the intestinal mucous cells, among others. Most probiotic bacteria belong to the LAB group but, nevertheless, it is generally known that probiotic features and benefits are extremely strain-dependent, even among LAB of the same species.

Some strains of Lactobacillus have showed certain activity against L. monocvtogenes infections. Regarding to this, the report of Kotsou et al, "In vitro Assessment of Probiotic Properties of Lactobacillus Strains from Infant Gut Microflora", Food Biotechnology 2008, Vol. 22, pp. 1-17, depicts different Lactobacillus species with probiotic properties and reports an in vitro activity against L. monocvtogenes. Although one of the Lactobacillus species is a strain of L. fermentum (named LF-B21), the data of the report do not allow correlating the activity against the pathogen with this strain. LF-B21 further carries the disadvantage that it poorly resists the bile salt environment. Moreover, Kotsou et al. clearly indicate that in order to have concluding data animal tests are required, that is, the in vitro behaviour is not a *sine qua non* that the in vivo assays succeed.

To face Y. enterocolitica infections one must mention the document of Frick et al., "Lactobacillus fermentum Attenuates the Proinflammatory Effect of Yersinia enterocolitica on Human Epithelial Cells", Inflamm. Bowel Disease - 2007, Vol. 13, pp. 83-90. This document shows a strain of L. fermentum (DSMZ 20052) reporting in vitro activity as an immunomodulatory and anti-inflammatory agent against a non virulent Yersinia enterocolitica strain when Y. enterocolitica infection occurs. Namely, the strain DSMZ 20052 has the ability to inhibit Y. enterocolitica-induced IL-8 secretion. The authors report that the anti-inflammatory effect is due to a soluble phospholipid produced by the strain of L. fermentum. Nonetheless, neither adhesion to nor invasion of Y. enterocolitica into HeLa cells was inhibited by co-infection with L. fermentum. It is finally concluded that this strain of L. fermentum may have probiotic properties modulating intestinal inflammatory responses, thus offering new therapeutic strategies, and that further tests in animal models should be performed.

Several documents depict strains of Lactobacillus and some L. fermentum show in vitro activity against wide spectra of pathogens, such as Listeria monocytogenes, Salmonella enteriditis, Salmonella typhimurium, Escherichia coli, Yersinia enterocolitica, Staphylococcus aureus, Shigella flexneri, Bacillus subtilis and Pseudomonas aeruginosa, among others. Examples of such documents are the reports of Bao et al., "Screening of potential properties of Lactobacillus fermentum isolated from traditional dairy products", Food Control 2010, Vol. 21, pp. 695-701; and the report of Mojgani et al., "Screening of locally Isolated Acid Bacteria for Use as Probiotics in Poultry in Iran", The Journal of Poultry Science 2007, Vol. 44, pp. 357-365. Nonetheless, one must mention that all these documents show in vitro data which cannot be directly extrapolated to in vivo data.

Thus, the art fails in providing L. fermentum strains with proved advantages and holding in vivo effects. Thus, making impossible their applicability in favour of the health by means of feasible formulations. Moreover, and despite the advances in the field of oral probiotics, it is clear from the above that new probiotic strains are needed which, having a wide spectrum of benefits in the intestinal tract or other organs, do not present adverse effects and allow the manufacture of feasible formulations.

### SUMMARY OF THE INVENTION

The inventors have isolated a new probiotic strain of L. fermentum that does not alter the organoleptic features of the edible products and also retains its probiotic activity in pleasant edible formulations, such as functional foods. Additionally, the strain of L. fermentum is highly active against gastrointestinal disorders or symptoms thereof. Further, the strain has advantageous immunomodulatory effects.

Thus, in a first aspect the invention provides a new isolated strain of Lactobacillus fermentum deposited at the Colección Espanola de Cultivos Tipo (CECT) under the accession number CECT 7472, on the 4^{th} of December 2008.

The strain of L. fermentum of the invention, isolated from goat milk (Capra hircus) was deposited, according to the Budapest Treaty, on the 4^{th} of December 2008 in the Colección Española de Cultivos Tipo (CECT) in the Universidad de Valencia C.P 46100 Burjasot, Valencia (Spain), by the depositor Mr. Agustín Lasserrot Cuadrado, sited at BIOT, Parque Tecnológico de Ciencias de la Salud, Avenida de la Innovación n°1, C.P 18100 Armilla, Granada (Spain). The strain of L. fermentum was identified by the depositor with the reference D3, and received the CECT accession number CECT 7472 after the International Authority of Deposit declared the strain as viable.

The depositor, Mr. Agustín Lasserrot authorised BIOTMICROGEN, S.L., sited in Parque Tecnológico de Ciencias de la Salud, Avda. de la Innovación 1, 18100 Armilla, Granada (Spain), to refer to the deposited biological material in the European patent application having the representative's reference number P1829EP00, and gave his unreserved and irrevocable consent to the deposited material being made available to the public as from the date of filing of this patent application.

The strain, object of the invention, is advantageous over the other prior art strains, since it is active against infections caused by virulent strains of enteropathogens Listeria monocytogenes and Yersinia enterocolitica. In fact, the Lactobacillus fermentum CECT 7472 is able to significatively shortening the colonization of the intestinal tract by a virulent Yersinia enterocolitica strain, thus promoting the elimination of yersiniae and the prompt healing of the diseases caused by said bacteria. On the other hand, the strain is able to clean the presence of the intracellular pathogen L. monocytogenes from vital organs, such as the spleen and the liver. Therefore, the diseases caused by Listeria are healed or prevented.

In the sense of this invention "intracellular pathogens" are microorganisms that are capable of growing and reproducing inside the cells of a host.

The term "enteropathogen" is to be understood as encompassing any microorganism which causes enteropathy or pathology in the intestine.

The above-mentioned advantages are of great relevance, but the strain of L. fermentum CECT 7472 also provides other interesting applications derived from the fact that it can be formulated as an edible product, and can be administered easily.

In a second aspect, the invention relates to a pure bacterial culture which comprises the L. fermentum CECT 7472.

This pure culture, as well as the strain itself, may be used in an edible product that comprises a nutritionally effective or sufficiently healthy amount of the strain, together with appropriate amounts of other edible ingredients.

The terms "effective amount" and "sufficiently healthy amount" as used herein mean an amount of an active agent high enough to deliver the desired benefit, but low enough to avoid side effects within the scope of medical judgment.

As mentioned above, the formulation of the strain of the invention as an edible product gives rise to attractive and pleasant compositions that may be administered in food. In this way, the strain produces its advantageous effects by consuming food.

The term "edible product" is used herein in its broadest meaning, including any type of product, in any type of presentation, which can be ingested by an animal, but excluding pharmaceutical and veterinary products.

Another aspect of the invention is therefore, a process for the preparation of such an edible product, and the L. fermentum CECT 7472 culture thereof in a suitable medium, such as a food substrate. The process comprises adding the strain in the product. The edible product may contain multiple edible ingredients or only one.

Moreover, the strain L. fermentum CECT 7472 maintains its probiotic features when included in the edible product.

The strain of Lactobacillus fermentum CECT 7472 is also for use as gastrointestinal tract health promoter.

A "gastrointestinal tract health promoter" is any ingredient which enhances the health of the gastrointestinal tract in terms of providing a good gastrointestinal life quality (digestion, absorption, defecation), which in turn is known to have a good impact in the whole life quality of an individual including the physical, psychological and social state. According to the World Health Organization, health is a state of whole social, physical and psychological well-being and not the mere disease absence.

The strain of L. fermentum CECT 7472 of the invention is in addition likely useful as a prophylactic and/or therapeutic agent, due to the capability of interfering with Listeria monocytogenes and Yersinia enterocolitica.

### BRIEF DESCRIPTION OF THE DRAWINGS

FIG. 1 is a graphic showing the logarithmic units (ULOG) of Y. enterocolitica detected in faeces of treated (square) and non-treated (triangle) mice in relation with the post-infection days (d).
FIG. 2 and FIG. 3 are graphics indicating the logarithm of colony formation units of L. monocytogenes per gram of tissue (Log CFU/g) in liver and spleen, respectively. Black bars are the control animals (non treated with L.fermentum); white bars are the treated animals (previously treated with L.fermentum); d3 means at day 3 post-infection; d6 means at day 6 post-infection.
FIG. 4 is a bar diagram depicting the number of different bacteria adhered per Caco-2 cell (N/c) in an adhesion assay performed with Y. enterocolitica O9 (Y. enterocolitica O9); L. fermentum CECT 7472 (L. fermentum) and Bacillus subtillis (B. subtillis).
FIG. 5 and FIG. 6 show the colony formation units (Log10 CFU) in spleen of BALB/c mice after several days (d) post-inoculation of L. fermentum CECT 7472 in control animals and immunodepressed animals, respectively.
FIG. 7 is a bar diagram showing the general physical state of some individuals who consumed goat milk containing 10⁷ CFU/ml of Lactobacillus fermentum CECT 7472. TO is time 0 or before the consumption; and T1 is time 1 or after the consumption. Y-axis values indicate the general physical state, which is a way to evaluate the satisfaction and gastrointestinal life quality of the individuals, according to GIQLI test.
FIG. 8 is a 1000x optical microscope image of Caco-2 cells. Arrows show the strain L.fermentum CECT 7472 adhered to the surface of the cells.

### DETAILED DESCRIPTION OF THE INVENTION

Although there have been various attempts to use probiotic strains for treating or preventing many diseases, there is a wide variability when the probiotics are used in the treatment of gastrointestinal disorders. It has now been found that the L. fermentum CECT 7472 is highly active against hazardous enteropathogens and intracellular pathogens, thus preventing and/or reducing the gastrointestinal symptoms caused by these.

When used as ingredient in an edible dairy product the strain of L. fermentum CECT 7472 is present in a range from 10⁹ to 10¹⁰ CFU/g (CFU-colony formation units).

The edible product is preferably selected from the group consisting of a milk product, a yogurt, a curd, a cheese, a fermented milk, a milk powder, a milk based fermented product, meat based fermented products, an ice-cream, a cereal based fermented product, a milk based powder, a beverage, a flour, a chewing-gum, a pet food, a dietary supplement, a functional food, a nutrition formula, and elderly an infant formulas.

Particularly interesting edible products are dietary supplements and infant formulas. In the sense of the present invention, dietary supplements also include nutraceuticals, which are known to be extracts of foods that have a nutritional effect on human health. Animal food is also included in the scope of the invention. The compositions of the invention could be also used as an ingredient in other food products.

As will be illustrated below, the strain of L. fermentum CECT 7472 is especially suitable for goat milk products, although it can be used in all types of milk. Thus, it is worth mentioning that a liquid or solid yogurt of goat milk with high organoleptic features may be made using the strain of the invention as additive together with common fermentative starters, such as Lactobacillus delbrueckii bulgaricum, Streptococcus thermophilus.

When the strain of L. fermentum CECT 7472 of the invention is employed as an ingredient of an edible product composition, said strain is preferably used in the form of a liquid in medium inoculums.

The examples below demonstrate that the strain is suitable for use in benefit of the gastrointestinal health, thus ameliorating gastrointestinal tract disorders or symptoms thereof. In the sense of the present invention the "gastrointestinal disorders" are diseases and syndromes of the different organs which constitute the gastrointestinal tract, and include diarrhoea, irritable bowel syndrome, inflammatory intestinal disease, abdominal pain, constipation, Crohn's disease, or bloating, Peyer's patch necrosis, chronic lymphadenopathy, gastroenteritis, and hepatic or splenic abscesses. All these diseases may have a bacterial aetiology or be the result of complex factors, such as the genetic predisposition of any individual, or others.

When the gastrointestinal disorders or associated symptoms come from enteropathogen infections, it has been determined that the strain L. fermentum CECT 7472 is especially useful when the pathogens are Y. enterocolitica and L. monocytogenes.

Thus, the strain L. fermentum CECT 7472 is especially useful as gastrointestinal tract health promoter when the tract is invaded by a pathogen selected from the group consisting of Yersinia enterocolitica and Listeria monocytogenes. In the same way, an edible product comprising the strain provides the same effect.

Further, another advantageous feature of the strain of the invention is its role as immune response modulator agent. Namely, the strain L. fermentum CECT 7472 promotes the production of the anti-inflammatory interleukin-10 (IL-10), up-regulates gamma interferon (IFN-γ), and consequently macrophage activation inhibits the growth of pathogens.

The cytokine production by spleen cells from L.fermentum treated and untreated mice response to B.cell and T-cell mitogens was measured by commercial by commercial ELISA assays.

Therefore, the strain L. fermentum CECT 7472 shows the ability to modulate per se the production of cytokines, which are messenger molecules that regulate the inflammatory and immune responses in the body. These immunomodulatory effects are beneficial to the host because they help to achieve an improved disease resistance and diminished risk of allergies. It is known that Gram negative bacteria living in the GIT show the molecule LPS (lipopolysaccharide) on their cell surface, which induces the production of inflammatory markers from the intestinal mucous cells. Lipopolysaccharides (LPS) constitute the antigen O and the Gram negative bacteria endotoxin, which play an important role in the pathogenesis of bacterial infections, as well as in the interaction with the host and its defence system. Probiotic supplementation with L. fermentum CECT 7472 in the form of an edible product, can change this situation to favour a greater presence of Gram positive bacteria in the GIT (including the lactic acid bacteria group), and increasing antagonistic properties against some Gram negative microorganisms and therefore reducing the presence of LPS in the GIT.

All these applications of the L. fermentum CECT 7472 in favour of the health of animals, including humans, then inspire one to manufacture functional food formulated with this effective strain such as yogurts or other fermented milk products.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### Example 1. Isolation of the strain and characterization

The initial isolation was carried out from goat milk on MRS agar (RENEL commercial brand). The screening was carried out using Gram staining and the catalase production test, discarding all those that were not gram-positive bacilli and catalase negative. After, a biochemical characterization was made of the selected strains and the major species were identified as mainly belonging to the genus Lactobacillus, Lactobacillus plantarum (42%) and Lactobacillus acidophilus (15%), and in a smaller amount, L. salivarius, L. casei, L. paracasei and L. fermentum. Once the strains were characterized, they were isolated in pure culture and preserved at 4°C for further studies.

The fermentation pattern of Lactobacillus D3 (CECT 7472) is the following:
- D-Ribose
- D-Galactose
- D-Glucose
- D-Mannose
- D-Maltose
- D-Lactose
- D-Raffinose
- Potassium D-gluconate

Example 2. Effect of the strain Lactobacillus fermentum CECT 7472 in an in vivo model of Yersinia enterocolitica infection.

A virulent Yersinia enterocolitica infection model was used to assess the resistance to enteropathogens. Two groups of six BALBC/c female mice, 20g weight each, were administered (via intraesophageal) during twelve days, one with the Lactobacillus fermentum CECT 7472, and the other (the control group) only administered with the vehicle.

After the treatment period of twelve days, the study and control batches both were inoculated with Yersinia enterocolitica 09, infecting them with the suspension at a 10⁸ CFU concentration of bacteria via intraesophageal with a rigid cannula. From then on, quantitative coprocultures were performed periodically, during fifteen days, on every animal to determine the amount of Yersinia per gram of feces. Daily, until the end of the experiment, animals were administered with a conventional dose of Lactobacillus fermentum CECT 7472.

The results showed that both batches were infected with the enteropathogen (being detected in feces from the first day after the inoculation). There were no significant differences between both counts during the first month but, finally, on day 50 after the infection all the animals treated with the Lactobacillus fermentum CECT 7472 were able to eliminate Y.enterocolitica, since yersiniae were not found in feces. The control group took 70 days to eliminate the bacteria, thus demonstrating that the effect of the Lactobacillus fermentum CECT 747 although not impeding the development of the infection, does act favorably to recuperate from and overcome the infection in a meaningful shorter period of time.

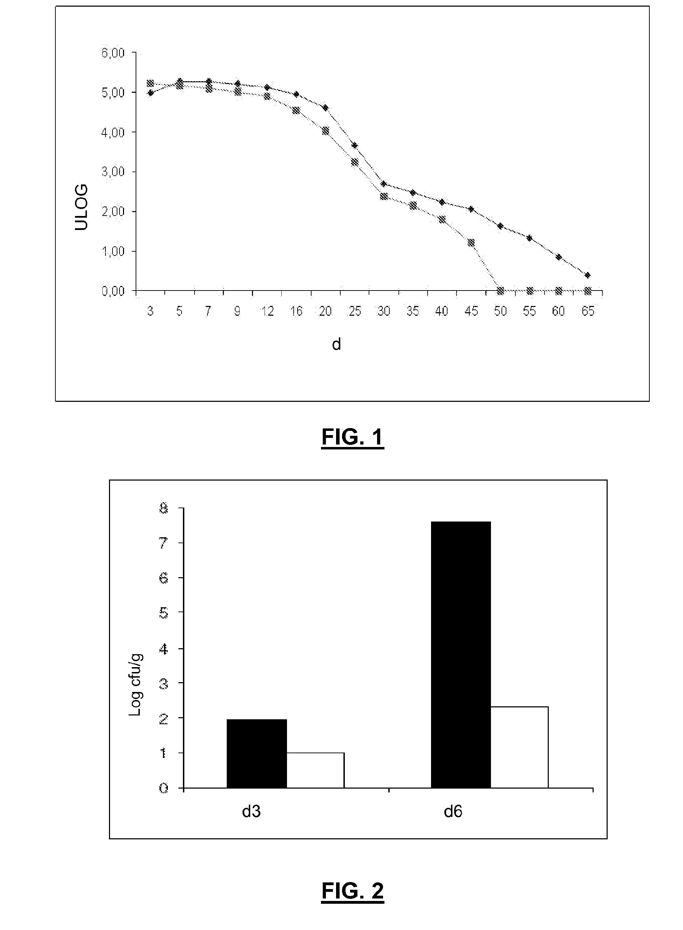

The results obtained in the above in vivo assay are depicted in FIG. 1, wherein the logarithm of the colony formation units of Y. enterocolitica in feces versus the post-infection day is shown. Square data correspond with the values obtained from the treated animals, and triangle data are the controls (not treated).

### Example 3. Effect of the strain L. fermentum CECT 7472 in an in vivo model of L. monocytogenes infection.

To demonstrate the resistance to intracellular pathogens a Listeria monocytogenes infection model was used. The same as in the Yersinia model (Example 2), two groups of six animals were assayed. One group was continuously administered with the selected Lactobacillus fermentum CECT 7472 for twelve days and the control group was only administered with the vehicle. After, both groups were inoculated with a Listeria monocytogenes suspension via intraesophageal.

This infection model induces a general sepsis in the animal after forty-eight hours. Thus, on the third and sixth day after the infection, half of each group was sacrificed by cervical dislocation. Livers and spleens were taken in all the cases and homogenized to be cultured in order to enumerate the infecting bacteria.

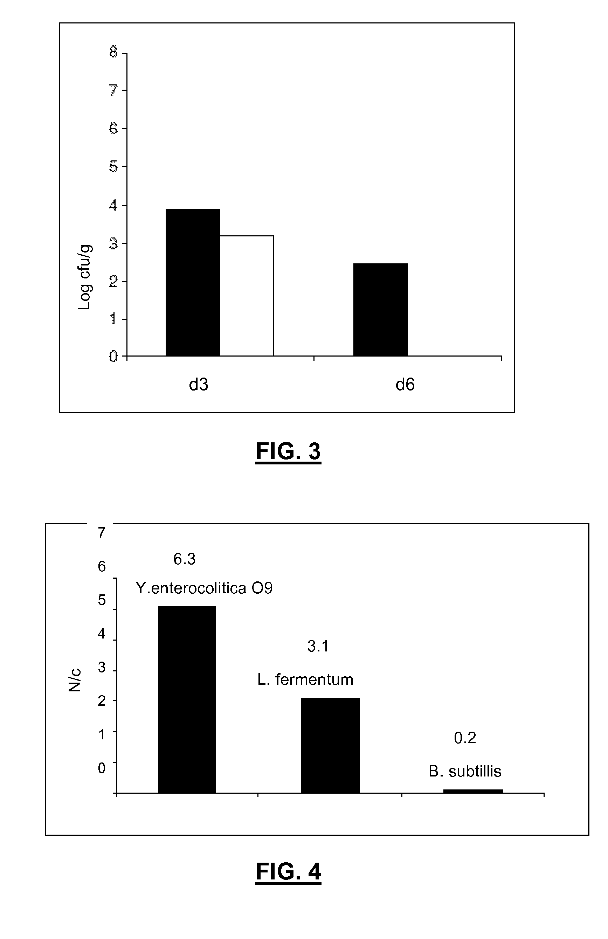

FIG. 2 and FIG. 3 are graphics indicating the logarithm of colony formation units of L. monocytogenes per gram of tissue (cfu/g) in liver and spleen, respectively, at day three or six post-infection of the mice. The data derivable from these figures clearly show that control animals (black bars) presented always higher levels of the microorganism than the treated animals. It is worth mentioning that at day six post-infection the spleen was free of the intracellular pathogen, indicating that the presence of the probiotic strain L. fermentum CECT 7472, acts as prophylactic agent avoiding or reducing the development of infections caused by L. monocytogenes.

### Example 4. Characterization of the probiotic properties of strain L. fermentum CECT 7472.

With the aim of determining the probiotic features of the new isolated strain of L. fermentum, several assays were performed that finally conclude that the strain CECT 7472 is a good probiotic.

It is important to note that each bacterial strain interacts with the GIT epithelium in a specific way. For this reason, when a probiotic strain is defined a strain specific citation is needed, rather than a species generalization. That is, the data from a specific strain are not directly extrapolated to the whole integers of the species itself.

### 4.1. Resistance to gastric acid and bile salts environment.

According to FAO guidelines, which are the guidelines emitted by Food and Agriculture Organization of the United Nations, probiotics used on food should survive on their way through the gastrointestinal tract and be able to grow in the bowel. So they must be resistant to gastric acid and be able to grow with bile salts. In addition, probiotics must have beneficial effects in the host.

The L. fermentum CECT 7472 strain was significantly resistant to gastric acid and was able to grow with high concentration of bile salts, thereby demonstrating its ability to pass through the gastric barriers and reach the colon to settle there.

### 4.1.1 Resistance to gastric acid environment.

A 10⁸ CFU/ml concentration of bacterial suspension from a 24 hour L.fermentum culture was treated with 0,15N HCL for 45 minutes to simulate the gastric conditions. After the incubation, decimal dilutions were plated on MRS agar and incubated at 37°C for 24-48 hours. A control group was prepared using sterile saline solution instead of HCl. Finally, the colonies were enumerated both in HCl and control conditions.

### 4.1.2 Bile salts resistance environment.

Decimal dilutions of a 12 hour L. fermentum CECT 7472 culture at a 10⁸ CFU/ml concentration were plated on MRS agar supplemented with different bile concentrations 0,05% to simulate the gastrointestinal conditions. The plaques were incubated at 37°C for 24-48 hours. A control group was prepared plating the dilutions on MRS agar without bile supplementation using sterile saline solution. Finally, the colonies were enumerated in all assayed conditions.

Table 1 illustrates the final colony formation units of L. fermentum CECT 7472 detected after the acid and bile treatments. A saline solution was used as a control.

**Table 1:**

| | Description | Count (CFU/mL) |
|---|---|---|
| HCL | Saline Solution (Control) | 2,8·10⁶ |
| | HCI 0.15 N | 8,0·10⁵ |
| Bile | Saline Solution (Control) | 2,9·10⁸ |
| | Bile 0.05 % | 1,3·10⁸ |

### 4.2. Effect on enteropathogens.

The L. fermentum CECT 7472 strain showed antimicrobial activity against different enteropathogens, both gram-positive and gram-negative.

In vitro assays showed inhibition of the following strains:
- Yersinia enterocolitica, strain IP383, serotype 09,
- Listeria monocytogenes ATCC 13932
- Salmonella entérica. Serovar Enteritidis, serotype London

One drop of a 10⁸ CFU/ml bacterial suspension from a 24 hour culture L. fermentum CECT 7472 was plated on the center of MRS agar petri plaques and incubated at 35°C for 24 hours. Then, TSA tubes were inoculated with different bacterial suspensions of Yersinia enterocolitica, Listeria monocytogenes, and Salmonella enteric Serovar enteritidis, at a 10¹⁰ CFU/ml concentration. After, they were poured over the previously plated plaques of the L. fermentum CECT 7472 strain. These plaques were incubated at 37°C for 24 hours and growth inhibition halos around L. fermentum CECT 7472 were measured.

Table 2 shows the inhibition halos in millimetres detected in the plaques for every assayed pathogen strain.

**Table 2:**

| Strains | Halo (mm) |
|---|---|
| Y. enterocolitica | 54 |
| L. monocytogenes | 65 |
| S. enterica | 30 |

As can be seen in Table 2, the strain L. fermentum CECT 7472 highly inhibits the growth of the pathogens.

### 4.3. Adhesion to Caco-2 cells.

The ability to adhere enterocyte surface is considered as a requeriment for probiotic bacteria.

To assess the adhesion ability to cellular lines Caco-2 cells were used. This cell line comes from human colon cancer. These cells show many characteristics of the intestinal epithelial human cells, having similar morphological and biochemical characteristics to enterocytes. Therefore, results from assays with Caco-2 cells will be able to be correlated with those of enterocytes human.

The cell line was cultured on single-layer and then incubated with the strain for 2 hours at 37°C and 5% CO2. After, the single-layer was fixed and stained with methylene blue.

The adhesion ability was done by counting the amount of bacteria adhered per cell in 10 consecutive fields using a microscope. As positive and negative control we used Yersinia enterocolitica 09 and Bacillus subtilis respectively. These results are shown in FIG.4.

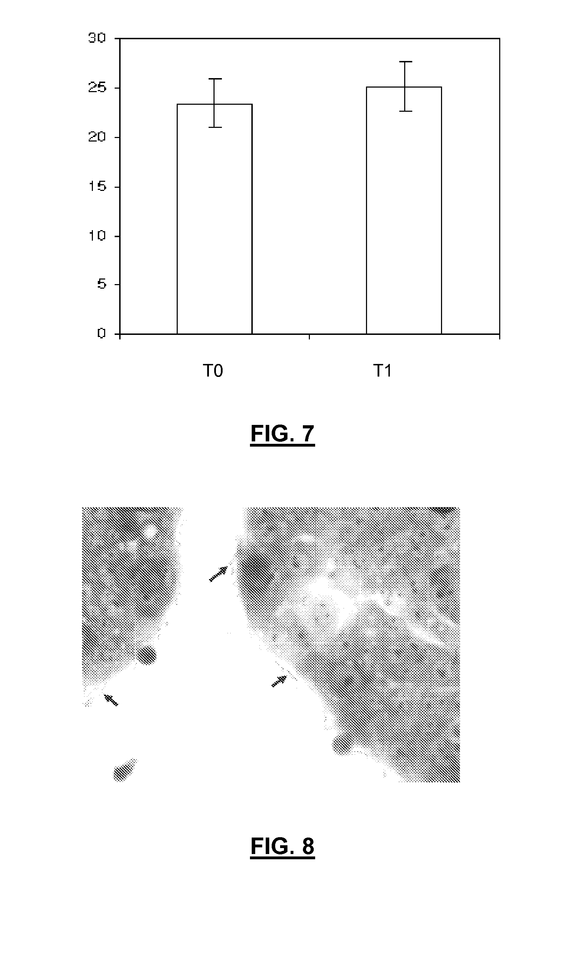

The results in FIG. 4, showing the number of adhered bacterial cells per Caco-2 cell, clearly reveal that the L. fermentum CECT 7472 of the invention adheres well to the intestinal cell model. Therefore, it can be concluded that L. fermentum CECT 7472 will adhere well to the epithelium colon cells of the individuals to whom the probiotic is administered. In FIG. 8 a 1000 x optical microscope image of the strain adhered to the cells is also showed.

### Example 5. Toxicity assays and bio security data.

The selected strain belongs to Lactobacillus genus, a genus that has been historically used on food fermentation, being commonly classified as safe, included in the international classification GRAS. Generally recognized as safe is a designation created by the organism Food and Drug Administration (FDA) in 1958. FDA awards GRAS categories to those substances or compounds considered safe for the health, and hence they do not reuire further evaluations.

However, biosecurity tests of L. fermentum CECT 7472 strain were performed, which are the following:

### 5.1. Production of harmful enzymes.

It is known that some probiotics, due to their metabolism, are able to produce harmful enzymatic activities, such as α-chymotrypsin, la β-glucoronidase, β-glucosidase and N-acetyl- β-glucosaminidase.

To detect the presence of these enzymes a commercial APIZYM-Biomerieux kit was used. This kit is a semi quantitative method to determine simultaneously up to 19 enzymatic activities. The L. fermentum CECT 7472 strain did not show any of those harmful enzymatic activities.

### 5.2. Non-infectivity on immunocompromised animals.

To check the bio-security of L. fermentum CECT 7472 20 BALB-c female mice, 20g weight each were used. Two batches were used, one immunocompetent and the other immunocompromised, the latter group by immunosupression. This treatment assures leucopenia in the animals for five days after the last injection. All the animals were innoculated with L. fermentum CECT 7472 and later the spleens were extracted in sterile conditions to be homogeneised in a sterile solution, from which decimal dissolutions were taken and later taken to a MRS medium (Man, Rogosa and Sharpe medium) for a later incubation at 37°C for 24 hours in order to finally ennumerate the colonies, expressed as viable bacteria per organ.

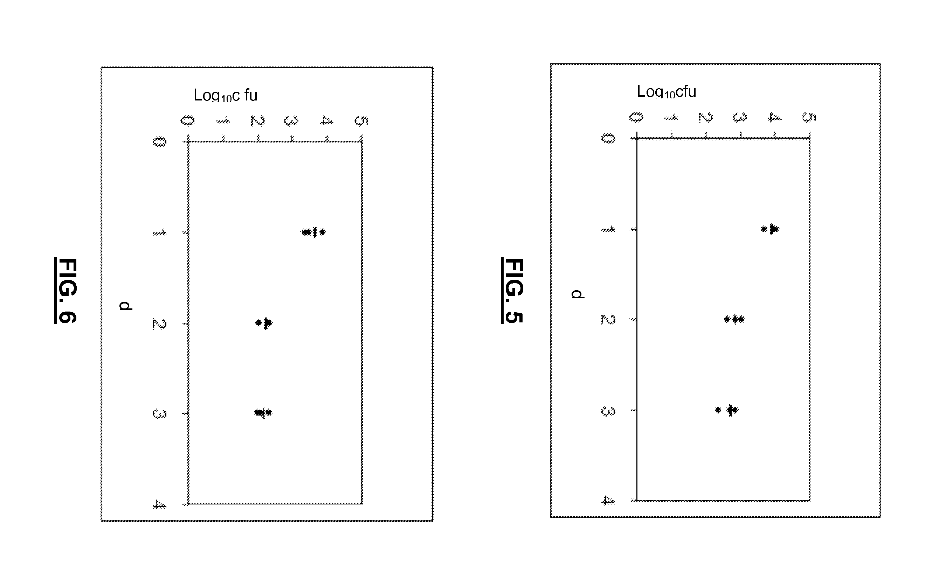

FIG. 5 depicts the corresponding data to the immunocompetent animals and FIG. 6 corresponds to the immunodepressed. These results verify that there is good bacteria depuration through the spleen, concluding that L. fermentum CECT 7472 is not harmful as a pathogenic microorganism in either immunocompetent or immunodepressed animals.

The data correspond to the result of three replicates.

FIG. 5 depicts the log₁₀ CFU of L. fermentum CECT 7472 versus the post-inoculation day of the isolated probiotic strain in immunocompetent mice. Analogously, in FIG. 6 the data of immunocompromised mice are illustrated.

### 5.3. Intestine barrier translocation

To demonstrate L. fermentum CECT 7472 is not capable of translocating the intestinal barrier an assay was carried out with twelve mice BALB-c immunosuppressed. These mice were administered 100 micro litres of L.fermentum CECT 7472 in a concentration of 10⁹ microorganisms viable per microlitre (µL) via intraesophageal during twelve days. After the treatment the animals were sacrificed, and their mesenteric nodes and spleens were extracted in sterile conditions later being homogenized in a sterile solution from which decimal dissolutions were taken and later taken to a MRS medium for a later incubation at 37°C for 24 hours in order to finally ennumerate the colonies, expressed as viable bacteria per organ. A lack of growth was observed in all the cultures obtained from all the samples of the animals, which indicates that L. fermentum CECT 7472 is not capable of passing through the intestinal barrier not being able to reach other parts of the body. Therefore it cannot produce systemic infection.

All these data taken as a whole clearly indicate that the new isolated strain of the invention is bio-safe, and it can be safely administered to a human or animal organism.

### Example 6. Immunomodulation activity

To assess the immunomodulator effect of the L. fermentum CECT 7472 strain, 40 BALB-c female mice of 20g weight were used. These animals were administered with the probiotics via intraesophageal for periods between 12 and 15 days.

### 6.1. Splenocyte proliferation assay

First, the splenocyte proliferation capability in presence of mitogen was studied. After administrating the probiotic, the splenic cells of the animals were cultured on microtitter plaques and incubated at 37°C, 5% CO2 and under saturated humidity for 72 hours in presence of two mitogenic agents, LPS (B-cell standard mitogen) and Concanavalin A (Con A, T-cell standard mitogen).

Cell proliferation was determined by a colorimetric method that measures MTT (tetrazolium derivative) reduction using a microELISA reader (ThermoLabsystems Multiskan EX), preset to read absorbance at 570 nm and 630nm. The results were expressed as arithmetic mean of the optical density.

The obtained results show that the administration of the probiotic led to a general increase in the immune response, stimulating the B-cells mitogen-mediated proliferation (LPS) and also the proliferation of T-cells (Concavalin A, Con A), however, due to the high standard deviation, the increase in Con A response was not statistically significant. Therefore, there is an immunopotentiation effect that may result in an increase in infections resistance.

### 6.2. In vivo evaluation of the immunomodulation ability of cytokine production by splenocytes.

To quantify cytokines, Thermo Scientific (Rockford, USA) ELISA kits with monoclonal mouse antibodies were used. The readings were performed using enzyme immunoassays from splenocytes culture supernatants from animals treated with the probiotic L. fermentum CECT 7472. As control a group of animals that received only the vehicle (sterile skimmed milk) were used. TNF-alpha, gamma interferon (IFN-γ) and interleukins IL-2, IL-4 y IL-10 were measured and, after the statistical analysis, a cell immunity increment was observed due to the increase in gamma interferon (IFN-γ) production, which acts by activating macrophages, making them more effective in destroying intracellular pathogens. This effect results in increased resistance to infection by these microorganisms. Also an anti-inflammatory effect was revealed because of the increase in IL-10, which coincided with a TNF-alpha decrease.

Some inflammatory intestinal diseases related to autoimmunity are associated with changes in intestinal bacterial microenvironment. The use of probiotics such as the L. fermentum CECT 7472 strain can help control the modulation of T-cells and B-cells, as well as other immunological mechanisms involved in the prevention of these diseases.

### Example 7. Edible goat milk fermented composition

The inventors have also developed several edible compositions, such as functional foods that are manufactured by adding in said compositions the strain L. fermentum CECT 7472.

In one alternative, the said strain is used as an additive with the usual fermentation starters, such as the combination Lactobacillus delbrueckii bulaaricus and Streptococcus thermophilus.

The lactic acid fermentation uses sugars from lactose hydrolysis (glucose and galactose) as substrates. The Lactobacillus bulgaricus and Streptococcus thermophilus bacteria transform lactose into lactic acid. During the fermentation process the structure of milk proteins are modified, resulting in the typical texture of the product, that it is why the choice and the amount of the selected starter as well as the physicochemical fermentation conditions are important in the process. Besides, lactic acid, a small amount of byproducts such as acetone, acetaldehyde, diacetyl and glucans are produced, substances that provide the typical odor and taste of the product.

A yogurt from goat milk was obtained by inoculating said milk with the common starters L. delbrueckii bulgaricus and S. thermophilus and after the fermentation process L. fermentum CECT 7472 was added as an additive in the amount of 10⁷ CFU/ml.

The milk inoculated with the starters was incubated during an average time of 4-5 hours, until reaching a final pH of approximately 4.7. Then, a rapid cooling until 4°C was done and finally bottled into 125 ml polyethylene containers suitable for food use.

Table 3 shows the physicochemical analysis data of two yogurt samples, as well as their fermentation time and the pH of the final product.

**Table 3.**

| Sample | Ferment. time (h) | final pH | Lactose (%) | Acidity (%) | Fat (%) | Ash (%) | Proteins (%) |
|---|---|---|---|---|---|---|---|
| Y2 | 5.50 | 4.29 | 3.04 | 0.85 | 2.88 | 0.78 | 3.68 |
| Y3 | 6.25 | 4.32 | 3.02 | 0.85 | 3.80 | 0.76 | 3.63 |

According to the current legislation on the pH of yogurt's it must be equal or lower to 4.6. Therefore, it is necessary to determine its value to verify compliance with the legislation. On the other hand, the acidity of yogurt indicates the percentage of lactic acid present. Both values must be correlated.

The values of the parameters shown in Table 3 were determined by the following techniques:
- Fat. Determination of fat by the Gerber method. International Rule of International Dairy Federation, FIL-IDF 22: 1963.
- Acidity. Determination of acidity by volumetry. Rule FIL-IDF-26: 1964 of the International Dairy Federation.
- Ash. Determination of dry extract. Rule FIL-IDF-21: 1962 of the International Dairy Federation.
- Lactose. Determination of lactose. Rule FIL-IDF-28: 1964 of the International Dairy Federation.
- Proteins. Determination of proteins by Kjeldahl method. Rule FIL-IDF-20: 1962 of International Dairy Federation.

Comparing both fermentative processes it was determined that the fermentation time of yogurt n°3 (Y3) was higher than in yogurt n°2 (Y2). A good correlation of the results, in terms of chemical composition, is shown in the table. The difference in fat amount between both samples was significant (0.92%) so it was determined that there was a direct relationship between the total amount of initial fat in the raw material and the average time of fermentation.

Another outstanding factor in the fermentative process is the right choice of fermentative microorganisms. To develop the fermented product, the inventors tested different starters from some commercial brands.

Fermentation processes were conducted using as starter the mixture S. thermophilus and L. delbrueckii bulgaricus (sample Y3 of Table 4), or the mixture S. thermophilus and L. delbrueckii lactis (Sample Y5 of Table 4)

**Table 4.**

| Sample | Ferment. time (h) | final pH | Acidity (%) | Fat (%) | Ash (%) |
|---|---|---|---|---|---|
| Y5 | 4.75 | 4.45 | 0.90 | 3.7 | 0.77 |
| Y3 | 6.25 | 4.32 | 0.85 | 3.8 | 0.76 |

Both L. delbrueckii ssp. Lactis and L. delbrueckii ssp. Bulgaricus result in products with similar organoleptic characteristics in terms of odor and taste, although the fermentations with L. delbrueckii ssp. Bulqaricus resulted in a more solid body of the product. That is why this inoculum was chosen to make solid yogurts.

In all fermentations, from similar milks in terms of chemical composition and fat content, fermentation times and pH values were very similar, which helped the process standardization and, therefore, the final product qualities.

With regard to the fermented goat milk product, it is worth mentioning the advantages of this kind of product with respect to other milk sources. Indeed, it has been widely accepted that goat milk in recent years is having a greater role in its consumption due to its nutritional and digestive properties. Many studies have documented that goat milk is more beneficial to health than cow's or sheep's milk. The fat globules are smaller and with a high content of medium chain fatty acids, which promotes their digestion and absorption. Their proteins are also digested more easily and their allergenic potential is lower. In addition, it has high phosphorous and bioavailable calcium content. Its consumption is specially recommended in digestive disorders, malabsorption syndrome and for young and elderly people.

But the beneficial effects of goat milk comparing with other milk sources do not stop here. It has also been documented that mineral absorption is higher, causing iron, which can be very useful to people with osteoporosis or anemia.

The edible fermented goat milk product of the present invention represents an interesting improvement over the marketed products in the field of functional foods. Indeed, it provides in a single product the benefits of fermented goat milk and the added benefits of incorporating a microorganism L. fermentum CECT 7472 that interacts advantageously with the immune system and the health in general.

### Example 8. Acceptability test.

In order to determine if the fermented milk product of Example 7 was feasible and had good organoleptic properties, several tests with volunteers were performed. The volunteers evaluated different yogurt samples including a goat milk yogurt of Example 7 (Y3), a commercial goat milk yogurt, an ecological commercial cow milk yogurt and an industrial commercial cow milk yog u rt.

The volunteers were asked about the taste, colour and texture of the yogurts. All the data were analysed by different methodologies and they affirm that the goat milk yogurt of Example 7 (Y3) was favourably accepted and 10% of the volunteers considered it as the best.

### Example 9. Test of gastrointestinal life quality in humans

A nutritional study was designed according to the Spanish Agency for Food Safety and Nutrition "Agencia Española de Seguridad Alimentaria y Nutrición (AESAN)" guidelines, the Nuremberg code, the collective agreement of Oviedo (Spain) and taking into account the Spanish law 15/1999 on the protection of personal data. Every volunteer signed an informed consent document which explains all the commitments and risks derived from the study, as well as their consent to participate freely without being remunerated.

According to the World Health Organization, health is a state of whole social, physical and psychological well-being and not the mere disease absence.

The volunteers were asked about the "gastric life quality". The test used to assess the gastric life quality of the studied volunteers was the so-called GIQLI survey, so the aim was to evaluate the physical and mental status of the volunteers before and after the treatment with the probiotic. In addition, this survey has been successfully used in some studies for the nutritional assessment of food and fermented dairy products on healthy individuals (V6zquez et al., "Application of GIQLI questionnaire to two groups of healthy yogurt consumers", Nutr Hosp - 2005, Vol.20, pp.420-428).

On a first visit, a survey of gastrointestinal quality was performed to all participants (test GIQLI), as well and as they were instructed to follow a standardized diet which included some food and medicine restrictions.

The first basal samples (time 0) were collected after a month of food restrictions, where the volunteers eliminated some kind of food from their diet such as yogurt, cheese and pickles as well as medicines like antacids and antibiotics. The purpose of this sample was to discard the presence of native populations of acid lactic bacteria in these individuals and also interference of medicine.

After these first basal samples, the volunteers started to take the probiotic fermented product. Specifically 200 ml/day of fermented goat milk at a concentration of 10⁷ CFU/ml of L. fermentum CECT 7472. The treatment with the probiotic was 30 days long.

The scores of GIQLI test were obtained at the end of two periods of the study: after the food restrictions period (time 0, T0) and after the period of fermented dairy product ingestion (time 1, T1 = 30 days). In most cases, the score at time 1 exceeded the score at time 0.

As can be seen from FIG. 7, which is a bar diagram showing the general physical state of the individuals at time 0 (T0) and at time 1 (T1), the satisfaction of the individuals was greater after the consumption of the goat milk containing 10⁷ CFU/ml of Lactobacillus fermentum CECT 7472.

The GIQLI test determines if the product has an influence in the gastrointestinal life quality and a good acceptability for the consumers in terms of digestion and defecation, as well as if it has a positive influence in the well-being general state. This test is an extensive test, useful to analyze several health aspects. It is a reproducible test, and a sensitive test, which means it is able to detect small health state variations.

The results state that the goat milk product with L. fermentum CECT 7472 had a positive influence in the gastrointestinal life quality. Moreover, the product was well tolerated by the individuals, who reported a good state of well-being after the ingestion of the product.

The analysis concluded that the individuals were felt better after the consumption of the probiotic strain of the invention.

All the examples listed herewith concluded that the strain L.fermentum CECT 7472, and the edible products comprising it are useful products to control the gut microflora, promoting health and life quality of individuals.

Although strains of L.fermentum have been previously reported for having in vitro activity against some enteropathogens, the present invention goes one step further by providing not only in vivo interesting data, but also giving a strain of L.fermentum that, due to its inherent properties, gives raise to improved results or effects over the L.fermentum strains of the prior art. Indeed, and as above indicated, the faculty of being able to be administered in the form of an edible product makes the strain L.fermentum CECT 7472, and all derivatives containing it, interesting products in the fields of food and nutrition industries and, in general, for health.

### REFERENCES CITED IN THE APPLICATION

- Kotsou et al, "In vitro Assessment of Probiotic Properties of Lactobacillus Strains from Infant Gut Microflora", Food Biotechnology 2008, Vol. 22, pp. 1-17.
- Frick et al., "Lactobacillus fermentum Attenuates the Proinflammatory Effect of Yersinia enterocolitica on Human Epithelial Cells", Inflamm. Bowel Disease, Vol. 13, pp. 83-90.
- Bao et al., "Screening of potential properties of Lactobacillus fermentum isolated from traditional dairy products", Food Control 2010, Vol. 21, pp. 695-701.
- Mojgani et al., "Screening of locally Isolated Acid Bacteria for Use as Probiotics in Poultry in Iran", The Journal of Poultry Science 2007, Vol. 44, pp. 357-365.
- Vázquez et al., "Application of GIQLI questionnaire to two groups of healthy yogurt consumers", Nutr Hosp-2005, Vol.20, pp.420-428.

## Claims

1. An isolated strain of Lactobacillus fermentum deposited at the Colección Española de Cultivos Tipo (CECT) under the accession number CECT 7472.

2. A bacterial pure culture which comprises the strain of claim 1.

3. An edible product which comprises a nutritionally effective amount of the strain as defined in claim 1, together with appropriate amounts of other edible ingredients.

4. The edible product according to claim 3, which is selected from the group consisting of a milk product, a yogurt, a curd, a cheese, a fermented milk, a powdered milk, a milk based fermented product, a meat based fermented product, an ice-cream, a cereal based fermented product, a beverage, a flour, a chewing-gum,a sweet, a sweet food, a pet food, a dietary supplement, a functional food, a clinical nutrition formula, a nutritional complement, a formula for the elderly and an infant formula.

5. A process for the preparation of an edible product as defined in any one of the claims 3-4, comprising adding the strain as defined in claim 1 or a culture as defined in claim 2 in said edible product.

6. The strain of Lactobacillus fermentum CECT 7472 as defined in claim 1 for use as a probiotic agent.

7. The strain of Lactobacillus fermentum CECT 7472 as defined in claim 1 for use as gastrointestinal tract health promoter.

8. The strain according to claim 7, as promoter of gastrointestinal tract health when the tract is invaded by a pathogen selected from the group consisting of Yersinia enterocolitica and Listeria monocytogenes.

9. An edible product as defined in any of claims 3-4 as a gastrointestinal tract health promoter.

10. The edible product of claim 9, which promotes the gastrointestinal tract health when the tract is invaded by a pathogen selected from the group consisting of Yersinia enterocolitica and Listeria monocytogenes.
